# EUROPEAN PATENT APPLICATION

(11) **EP 0 908 436 A1**
(43) Date of publication of application: **14.04.1999**
(21) Application number: 97915695.7
(22) Date of filing: 10.04.1997
(51) Int. Cl.: C07C 11/02, C07C 2/34, B01J 2/26, B01J 27/10, B01J 31/14, B01J 31/22

(54) **PROCESS FOR THE DIMERIZATION OF LOWER OLEFINS**

(30) Priority: 10.04.1996 JP 88094/96
(71) Applicant: Kyowa Yuka Co., Ltd., Tokyo 100 (JP)
(72) Inventor: FUKUDA, Yukitoshi, Yokkaichi-shi, Mie 510 (JP); HASHIMOTO, Keiichi, Yokkaichi-shi, Mie 510 (JP); MATSUSHITA, Shoshiro, Yokkaichi-shi, Mie 512 (JP)
(74) Representative: Casalonga, Axel
(86) International application number: JP9701231
(87) International publication number: WO9737954

(57) **Abstract**

The present invention relates to a process for dimerizing lower olefins characterized by the use of a catalyst comprising the following three components: (1) at least one salt or complex of a transition metal belonging to the platinum group of the periodic table, (2) at least one organoaluminum compound and (3) at least one inorganic metallic compound.

The invention provides a process for dimerizing lower olefins with very high yield and selectivity and also with very high yield per unit weight of catalyst. Also provided is a catalyst to be used in said process.

## Description

### Technical Field

The present invention relates to a process for dimerizing lower olefins which are obtained easily and in large quantities in the petrochemical industry to obtain higher olefins, and to a catalyst to be used in said process. The obtained higher olefins are important raw materials for plasticizers, etc.

### Background Art

As for the dimerization of lower olefins, techniques based on Ziegler's Nickel Effect have been widely known. For example, their details are described in "Organometallic Chemistry - Fundamentals and Applications-" (Akio Yamamoto, Shokabo, 1983, pp. 275 and 282-285). Japanese Published Unexamined Patent Application No. 92091/79 and Japanese Published Unexamined Patent Application No. 15751/79 disclose dimerization processes wherein a fatty acid salt of nickel and ethylaluminum dichloride are used together as catalysts. According to the above processes, olefins can be dimerized under mild conditions, but high boiling point by-products are formed along with the desired products and the selectivity for dimers is not high.

In order to enhance the selectivity for dimers, catalysts which are prepared by addition of various additives to Ziegler type catalysts are used. Japanese Published Unexamined Patent Application No. 136236/80 and Japanese Published Unexamined Patent Application No. 210034/84 disclose dimerization reactions using polyol-added catalysts. By these dimerization reactions, dimers can be obtained more selectively than by the dimerization reaction using a conventional Ziegler type of catalyst. However, there is still room for improvement in the above reactions in respect of the amount of high boiling point by-products.

Japanese Published Unexamined Patent Application No. 167932/82 discloses dimerization reaction using a catalyst to which a phosphorus compound and water were added, Japanese Published Unexamined Patent Application No. 169433/82 discloses dimerization reaction using a catalyst to which a phosphorus compound and dialuminoxane were added, and Japanese Published Unexamined Patent Application No. 221335/89 discloses dimerization reaction using a catalyst to which a phosphorus compound and an active hydrogen compound were added. However, these processes are not suitable for industrial application in view of the facts that they are not satisfactory in respect of the selectivity for the desired products and that they are applicable only to α-olefins, which are not readily available in a pure form for industrial use.

Japanese Published Unexamined Patent Application No. 41440/92 discloses dimerization of olefins using a catalyst prepared by adding a quaternary phosphonium salt to a Ziegler type of catalyst, but the process is still unsatisfactory in respect of the amount of high boiling point by-products.

Japanese Published Unexamined Patent Application No. 228016/94 and Japanese Published Unexamined Patent Application No. 92134/96 disclose techniques relating to dimerization of lower olefins using specific bisphosphite ligands. However, these processes are not entirely satisfactory in that the ligands to be used are specific and the proportion of the formed by-products which are oligomers except dimers to the desired dimers is high.

Japanese Published Unexamined Patent Application No. 27037/96 discloses that the selectivity for dimers is enhanced by the use of a catalyst system comprising a conventional Ziegler type of catalyst and an organoaluminum compound such as methylaluminoxane. However, there is room left for improvement in this process in respect of yield.

Apart from the dimerization processes using the above homogeneous catalysts, there are also known techniques relating to the dimerization of lower olefins using heterogeneous catalysts such as fixed catalysts and metal catalysts supported on carriers.

For example, there have been reports on the dimerization using, as a catalyst, a metal oxide such as zirconia or titania (Japanese Published Unexamined Patent Application No. 213781/93), the dimerization using a Ziegler type of catalyst carried on an acidic metal oxide (Japanese Published Unexamined Patent Application No. 142125/87) and the dimerization using, as a catalyst, a combination of a noble metal such as palladium or ruthenium and a specific acid (Japanese Published Unexamined Patent Application No. 145126/86).

Japanese Published Unexamined Patent Application No. 278355/86 discloses a technique relating to oligomerization of olefins using a metal catalyst supported on a carrier. This technique relates to oligomerization of olefins using a metal catalyst supported on a carrier which is prepared from a metallic compound supported on a porous carrier and an activating liquid mainly comprising an oganoaluminum compound and a halogenated aluminum compound.

These heterogeneous catalysts are industrially advantageous in that the separation thereof from the formed products is very easy, but are disadvantageous compared with homogeneous catalysts because it is generally difficult to prepare them with high reproducibility and the yield of the desired products per unit weight of catalyst is low. Therefore, industrial application of heterogeneous catalysts will be difficult due to economic restrictions, unless they have considerably long lives. Particularly, in the case of oligomerization of olefins where polymeric by-products are apt to be formed, application of the heterogeneous catalysts is often difficult because the polymeric by-products attach to the catalysts and lower the activity thereof. Further, these conventional dimerization techniques are not satisfactory in respect of the selectivity for the desired products.

As another example of heterogeneous catalyst, Japanese Published Unexamined Patent Application No. 24280/97 discloses a technique relating to a novel caralyst composition for two-phase catalysis and a process for the oligomerization of olefins using this novel catalyst composition. This composition is a heterogeneous catalyst comprising lithium halide, hydrocarbylaluminum halide and catalyst compound such as a nickel compound, and the phase of a catalyst and the phase of olefin as a starting material make up two separated phases which may be liquid-liquid or solid-liquid.

In the dimerization of lower olefins using this heterogeneous catalyst, the problems relating to the separation of a catalyst from the formed products which are usually found in the dimerization using a homogeneous catalyst have been solved, but the yield of dimers per unit weight of catalyst is unsatisfactory. Particularly, the yield per unit weight of hydrocarbylaluminum is low because a large amount of hydrocarbylaluminum is used to form the heterogeneous catalyst. Therefore, dimerization using this catalyst is industrially disadvantageous, unless the formed heterogeneous catalyst is fully recycled.

So far, no process has been known for dimerizing lower olefins with high yield of and high selectivity for dimers which are the desired products and also with high yield per unit weight of catalyst.

### Disclosure of the Invention

The present invention relates to a catalyst which is obtained by mixing (1) at least one salt or complex of a transition metal belonging to the platinum group of the periodic table, (2) at least one organoaluminum compound and (3) at least one inorganic metallic compound. The invention also relates to a process for dimerizing lower olefins which is characterized by the use of said catalyst.

The present invention is described in detail below.

The catalyst of the present invention can be prepared by mixing (1) at least one salt or complex of a transition metal belonging to the platinum group of the periodic table, (2) at least one organoaluminum compound and (3) at least one inorganic metallic compound, at a temperature of -50 to 200°C, preferably 0 to 100°C. The catalyst can be prepared more stably in the presence of lower olefins to be dimerized. It is more preferable from the standpoint of operation to mix at least one organoaluminum compound and at least one inorganic metallic compound at a temperature of -50 to 200°C, preferably 0 to 100°C, and then add the resulting mixture to lower olefins to be dimerized in the presence of at least one salt or complex of a transition metal belonging to the platinum group of the periodic table at a temperature of -50 to 200°C, preferably 0 to 100°C. In this case, the reaction system becomes homogeneous and the catalytic reaction proceeds as a homogeneous catalytic reaction.

The term lower olefin as used herein refers to monoolefins having 2-7 carbon atoms such as ethylene, propylene, 1-butene, isobutene, 2-butene, 1-hexene and 1-heptene. Preferred monoolefins are those having 4 carbon atoms, for example, 1-butene, *cis* and *trans* 2-butene, isobutene and mixtures thereof, which are obtained in large quantities in the thermal cracking process or the fluid bed catalytic cracking process in the petrochemical industry and of which the dimerization products are useful as raw materials for plasticizers. The scope of the present invention includes dimerization of one of the above olefins and codimerization of a mixture of any two or more of the above olefins in any ratio.

The salts and complexes of the transition metals belonging to the platinum group of the periodic table to be used in the present invention include salts and complexes of platinum, palladium and nickel. These compounds are used in an amount of 10⁻⁷ to 10⁻³, preferably 10⁻⁵ to 10⁻³, more preferably 10⁻⁵ to 10⁻⁴ in the molar ratio based on olefins as a starting material. They may be employed alone or as a mixture of two or more kinds of compounds, and can also be employed in the form of solutions in aprotic solvents such as toluene, hexane, tetrahydrofuran, dimethoxyethane and dimethylformamide.

The scope of the present invention also includes the use of the salt or complex of a transition metal belonging to the platinum group of the periodic table supported on carriers such as porous substances and metal oxides.

Examples of the salts of platinum are platinum chloride, platinum bromide, platinum iodide, platinum acetate, platinum 2-ethylhexanoate, platinum naphthenate and platinum nitrate. Examples of the complexes of platinum are complexes of platinum salts such as platinum chloride and platinum bromide with acetonitrile, triphenylphosphine, tributylphosphine, 1,2-bisdiphenylphosphinoethane and 1,4-bisdiphenylphosphinobutane, dicyclopentadienylplatinum and platinum acetylacetonate.

Examples of the salts of palladium are palladium chloride, palladium bromide, palladium iodide, palladium acetate, palladium 2-ethylhexanoate, palladium naphthenate and palladium nitrate. Examples of the complexes of palladium are complexes of palladium salts such as palladium chloride and palladium bromide with acetonitrile, triphenylphosphine, tributylphosphine, 1,2-bisdiphenylphosphinoethane and 1,4-bisdiphenylphosphinobutane, dicyclopentadienylpalladium and palladium acetylacetonate.

Examples of the salts of nickel are nickel chloride, nickel bromide, nickel iodide, nickel acetate, nickel 2-ethylhexanoate, nickel naphthenate and nickel nitrate. Examples of the complexes of nickel are complexes of nickel salts such as nickel chloride and nickel bromide with acetonitrile, triphenylphosphine, tributylphosphine, 1,2-bisdiphenylphosphinoethane and 1,4-bisdiphenylphosphinobutane, dicyclopentadienylnickel and nickel acetylacetonate.

A preferred transition metal belonging to the platinum groups of the periodic table is nickel, and as the salt or complex thereof, nickel 2-ethylhexanoate is preferred.

Examples of the organoaluminum compounds are trialkylaluminum, dialkylaluminum halides, alkylaluminum dihalides, triarylaluminum, diarylaluminum halides, arylaluminum dihalides, dialkylaluminum alkoxides, alkylaluminum dialkoxides, dialkylaluminum alkylsulfides, alkylaluminum dialkylsulfides, dialkylaluminum hydrides, alkylaluminum dihydrides and aluminoxane. Preferred are trialkylaluminum, dialkylaluminum halides and alkylaluminum dihalides. These compounds may be used alone or in combination.

In said organoaluminum compounds, the alkyl and the alkyl moiety of the alkoxide and the alkylsulfide mean an alkyl group having 1-6 carbon atoms such as methyl, ethyl, propyl, isopropyl, butyl, isobutyl, t-butyl, pentyl and hexyl. They may be the same or different when their number is more than two. The aryl means an aryl group having 6-10 carbon atoms such as phenyl, tolyl and naphthyl. The halogen includes chlorine and bromine. The aluminoxane includes polymethyl aluminoxane, polyethyl aluminoxane, tetramethyl bisaluminoxane and tetraethyl bisaluminoxane.

Examples of the trialkylaluminum compounds are trimethylaluminum, triethylaluminum, triisobutylaluminum and trihexylaluminum. Examples of the dialkylaluminum halides are dimethylaluminum chloride, diethylaluminum chloride, dipropylaluminum chloride, diisobutylaluminum chloride, dihexylaluminum chloride, dimethylaluminum bromide, diethylaluminum bromide and diisobutylaluminum bromide. Examples of the alkylaluminum dihalides are methylaluminum dichloride, ethylaluminum dichloride, propylaluminum dichloride, isobutylaluminum dichloride, hexylaluminum dichloride, methylaluminumdibromide, ethylaluminumdibromide and isobutylaluminum dibromide.

Preferred organoaluminum compounds are alkylaluminum dihalides, particularly ethylaluminum dichloride.

When the salt or complex of a transition metal belonging to the platinum group of the periodic table and the organoaluminum compound are provided in the form of solutions in aprotic solvents such as hexane, heptane, benzene, toluene, xylene and tetrahydrofuran, they may be used as such.

The organoalkylaluminum compound is used in an amount of 10⁻⁶ to 10⁻¹, preferably 10⁻⁵ to 10⁻¹, more preferably 5 x 10⁻⁵ to 10⁻² in the molar ratio based on olefins as a starting material.

The proportion of the organoaluminum compound to the salt or complex of a transition metal belonging to the platinum group of the periodic table affects the yield of and the selectivity for the product. The amount of the organoaluminum compound to be used is preferably 50 or less, more preferably in the range of 5 to 30, particularly 10 to 25 in the molar ratio based on the amount of the salt or complex of a transition metal belonging to the platinum group of the periodic table. The use of the organoaluminum compound in an amount of more than 50 in the molar ratio will lower the yield and the selectivity, instead of enhancing them. The use of the organoaluminum compound in an excessive amount is undesirable also from the standpoints of economy and effective utilization of resources.

The inorganic metallic compounds employed in the present invention include various metals, and oxides, salts and complexes thereof. Preferred metals are aluminum, titanium, zirconium, magnesium, zinc, sodium, lithium, lanthanum and calcium. Examples of the salts of said metals are halides such as fluoride, chloride, bromide and iodide, addition salts of organic acid such as acetate, butyrate and 2-ethylhexanoate, addition salts of inorganic acid such as carbonate and sulfate, and hydroxide. Examples of the complexes are acetylacetonate complex, acetonitrile complex and triphenylphosphine complex.

Preferred inorganic metallic compounds are halides, particularly chloride, of aluminum, magnesium, zinc, lithium and calcium.

The inorganic metallic compound is used in an amount of 10⁻⁴ to 10, preferably 10⁻² to 1, more preferably 5 x 10⁻² to 5 x 10⁻¹ in the molar ratio based on the total aluminum atoms contained in the organoaluminum compound. The metallic compound may be used alone or as a mixture of two or more kinds of compounds.

The reaction temperature for the dimerization of lower olefins according to the present invention is in the range of 0 to 150°C, preferably 20 to 100°C. Generally, as the reaction temperature is raised, the rate of reaction is increased. However, it is not desirable that the reaction temperature is too high because the reaction pressure becomes high and the selectivity for dimers is lowered. The reaction pressure is not subject to any specific restriction and varies depending on the composition and the temperature of the system. The reaction pressure is usually in the range of 0 to 30 atm. gauge, preferably 1 to 10 atm. gauge. When the reaction is carried out by batch operation, the ratio of lower olefins as a starting material decreases and that of dimers having a higher boiling point increases as the reaction proceeds, and so the reaction pressure is usually lowered with the passage of time. The reaction time is 30 minutes to 20 hours, preferably 1 to 10 hours. As the reaction time becomes longer, the conversion of lower olefins as a starting material is raised, but the selectivity for dimers tends to decrease a little.

After the reaction is completed, the active catalyst is inactivated by addition of an aqueous solution of alkali such as sodium hydroxide and the desired dimers can be isolated from the separated oil layer according to conventional methods in organic synthetic chemistry such as distillation and various kinds of chromatography.

In carrying out the dimerization, inert compounds, for example, hydrocarbons such as methane, ethane, propane, butane, isobutane, hexane, heptane, toluene and xylene may be present in the reaction system. A solvent is not essential for the reaction, but the above hydrocarbons may be used as a solvent.

### Best Modes for Carrying Out the Invention

The present invention is described in more detail by the following Examples, Comparative Examples and Reference Examples.

Identification of the products and the by-products was carried out by comparison with standard by means of gas chromatography. Quantitative determination of the products and the by-products was carried out according to the internal standard method using gas chromatography. The gas chromatography was carried out using GC-14A (Shimadzu Corporation) and a glass column packed with PEG-HT packing.

### Example 1

In an autoclave which had been completely dried with heating and nitrogen-substituted were put nickel 2-ethylhexanoate (a 2.24 wt% nickel solution in hexane, 75.6 mg) and a butene mixture (21 g, composition: 15.6 wt% butane, 2.7 wt% isobutane, 35.1 wt% 1-butene, 35.5 wt% 2-butene, 11.1 wt% isobutene), followed by stirring for 5 minutes. To the resulting mixture was added 0.4 ml of treated ethylaluminum dichloride (hereinafter ethylaluminum dichloride is abbreviated as EADC) prepared by adding aluminum chloride to EADC according to Reference Example 1. The temperature inside the autoclave was raised to 45°C in an oil bath, and the mixture was subjected to reaction with stirring for 2 hours.

The pressure inside the autoclave was 2.9 atm. gauge at the start of reaction, but lowered to 2.3 atm. gauge in 2 hours.

After the completion of reaction, the pressure inside the autoclave was released and the resulting gas components were collected using a dry ice-acetone trap and analyzed by gas chromatography. The total amount of the components was 9.81 g and most of them were butanes and butenes. The autoclave was then opened and the solution therein was transferred into a beaker, followed by addition of 20 ml of a 0.5 N aqueous solution of sodium hydroxide to inactivate the catalyst. The oil layer was separated, and 10.8 g of the reaction product was obtained. Quantitative analysis of the reaction product by gas chromatography revealed that 9.0 g of octenes, which is dimers, was formed by the reaction. The conversion of butenes was 64%, and the selectivity for octenes was 84%. The octenes:dodecenes(trimers):hexadecenes (tetramers) ratio was 95:4:1. The yield per unit weight of catalyst was 5325 kg-octene/kg-nickel.

### Examples 2-3

Dimerization of the butene mixture was carried out in the same manner as in Example 1 under the conditions shown in Table 1.

The results are shown in Table 2.

**Table 1**

| Example | Butene | Nickel octylate* | Treated EADC | Reaction temp. | Reaction time |
|---|---|---|---|---|---|
| 2 | 21 g | 77.2 mg | 0.5 ml | 45°C | 2 hours |
| 3 | 21 g | 77.1 mg | 0.4 ml | 45°C | 7 hours |

| | | | | | |
|---|---|---|---|---|---|
| * Nickel octylate means nickel 2-ethylhexanoate. | | | | | |

**Table 2**

| Example | Conversion of butene | Yield of octene | Yield per unit wt. of catalyst (Ni)*^{a} | Yield per unit wt. of catalyst (EADC)*^{b} | Octene: dodecene: hexadecene |
|---|---|---|---|---|---|
| 2 | 66% | 50% | 4848 | 131 | 91:5:3 |
| 3 | 77% | 60% | 5864 | 158 | 91:6:3 |

| | | | | | |
|---|---|---|---|---|---|
| *^{a} Yield of octene obtained per kg of nickel atoms (kg) | | | | | |
| *^{b} Yield of octene obtained per kg of EADC (kg) | | | | | |

### Examples 4-11

Dimerization of the butene mixture was carried out in the same manner as in Example 1 under the conditions shown in Table 3 using treated EADC prepared in the same manner as in Reference Example 1 except that each of the metallic compounds shown in Table 3 was used in place of aluminum chloride.

The results are shown in Table 4.

**Table 3**

| Example | Metallic compound | Butene | Nickel octylate* | Treated EADC | Reaction temp. | Reaction time |
|---|---|---|---|---|---|---|
| 4 | ZnCl₂ | 21 g | 78.7 mg | 0.4 ml | 45°C | 2 hours |
| 5 | ZnCl₂ | 21 g | 71.7 mg | 0.4 ml | 45°C | 7 hours |
| 6 | LiCl | 20 g | 75.2 mg | 0.4 ml | 45°C | 2 hours |
| 7 | LiCl | 20 g | 74.7 mg | 0.4 ml | 45°C | 7 hours |
| 8 | MgCl₂ | 21 g | 72.4 mg | 0.5 ml | 45°C | 2 hours |
| 9 | MgCl₂ | 20 g | 78.4 mg | 0.5 ml | 45°C | 7 hours |
| 10 | CaCl₂ | 20 g | 79.9 mg | 0.4 ml | 45°C | 2 hours |
| 11 | CaCl₂ | 20 g | 78.2 mg | 0.4 ml | 45°C | 7 hours |

| | | | | | | |
|---|---|---|---|---|---|---|
| * Nickel octylate means nickel 2-ethylhexanoate. | | | | | | |

**Table 4**

| Example | Conversion of butene | Yield of octene | Yield per unit wt. of catalyst (Ni)*a | Yield per unit wt. of catalyst (EADC)*b | Octene: dodecene: hexadecene |
|---|---|---|---|---|---|
| 4 | 53% | 44% | 4347 | 118 | 96:3:1 |
| 5 | 74% | 62% | 6701 | 181 | 94:4:1 |
| 6 | 63% | 57% | 5590 | 151 | 95:3:0 |
| 7 | 79% | 70% | 6897 | 186 | 95:4:0 |
| 8 | 66% | 50% | 5115 | 138 | 91:6:3 |
| 9 | 85% | 67% | 6203 | 168 | 92:6:2 |
| 10 | 64% | 53% | 4818 | 130 | 97:3:0 |
| 11 | 76% | 62% | 5816 | 157 | 94:5:1 |

| | | | | | |
|---|---|---|---|---|---|
| *^{a} Yield of octene obtained per kg of nickel atoms (kg) | | | | | |
| *^{b} Yield of octene obtained per kg of EADC (kg) | | | | | |

### Examples 12-17

Dimerization of the butene mixture was carried out in the same manner as in Example 1 under the conditions shown in Table 5 using treated EADC prepared in the same manner as in Reference Example 1 except that each of the mixtures of metallic compounds shown in Table 5 was used in place of aluminum chloride and a 50% EADC solution in toluene was used in place of the 20% EADC solution in toluene.

The results are shown in Table 6.

**Table 5**

| Example | Metallic compound*^{a} | Butene | Nickel octylate*^{b} | Treated EADC | Reaction temp. | Reaction time |
|---|---|---|---|---|---|---|
| 12 | AlCl₃(5) | 20 g | 75.5 mg | 0.12 ml | 45°C | 2 hours |
| | LiCl(15) | | | | | |
| 13 | AlCl₃(5) | 20 g | 77.5 mg | 0.12 ml | 45°C | 7 hours |
| | LiCl(15) | | | | | |
| 14 | MgCl₂(5) | 20 g | 76.7 mg | 0.11 ml | 45°C | 2 hours |
| | LiCl(15) | | | | | |
| 15 | MgCl₂( 5) | 20 g | 81.5 mg | 0.11 ml | 45°C | 7 hours |
| | LiCl(15) | | | | | |
| 16 | CaCl₂(5) | 20 g | 73.2 mg | 0.12 ml | 45°C | 2 hours |
| | LiCl(15) | | | | | |
| 17 | NaCl(5) | 20 g | 74.6 mg | 0.13 ml | 45°C | 2 hours |
| | LiCl(15) | | | | | |

| | | | | | | |
|---|---|---|---|---|---|---|
| *^{a} The figures in ( ) indicate equivalents of the metallic compounds based on EADC expressed in mol%. | | | | | | |
| *^{b} Nickel octylate means nickel 2-ethylhexanoate. | | | | | | |

**Table 6**

| Example | Conversion of butene | Yield of octene | Yield per unit wt. of catalyst (Ni)*^{a} | Yield per unit wt. of catalyst (EADC)*^{b} | Octene: dodecene: hexadecene |
|---|---|---|---|---|---|
| 12 | 73% | 66% | 6571 | 178 | 95:4:1 |
| 13 | 88% | 79% | 7700 | 208 | 94:5:1 |
| 14 | 75% | 66% | 6452 | 177 | 95:4:1 |
| 15 | 88% | 78% | 6868 | 186 | 93:6:1 |
| 16 | 77% | 65% | 6514 | 176 | 94:5:1 |
| 17 | 66% | 62% | 6373 | 172 | 95:4:1 |

| | | | | | |
|---|---|---|---|---|---|
| *^{a} Yield of octene obtained per kg of nickel atoms (kg) | | | | | |
| *^{b} Yield of octene obtained per kg of EADC (kg) | | | | | |

### Comparative Examples 1-3

Dimerization of the butene mixture was carried out under the conditions shown in Table 7 in the same manner as in Example 1 except that EADC was used in place of the treated EADC prepared by addition of aluminum chloride in Reference Example 1.

The results are shown in Table 8.

**Table 7**

| Comparative Example | Butene | Nickel octylate* | EADC | Reaction temp. | Reaction time |
|---|---|---|---|---|---|
| 1 | 20 g | 62.5 mg | 0.5 ml | 45°C | 2 hours |
| 2 | 20 g | 116.1 mg | 0.5 ml | 45°C | 2 hours |
| 3 | 20 g | 197.7 mg | 0.5 ml | 45°C | 2 hours |

| | | | | | |
|---|---|---|---|---|---|
| * Nickel octylate means nickel 2-ethylhexanoate. | | | | | |

**Table 8**

| Comparative Example | Conversion of butene | Yield of octene | Yield per unit wt. of catalyst* | Octene: dodecene: hexadecene |
|---|---|---|---|---|
| 1 | 18% | 1% | 2.4 | 45:12:44 |
| 2 | 32% | 15% | 20.7 | 78:11:11 |
| 3 | 44% | 39% | 32.4 | 83:10:7 |

| | | | | |
|---|---|---|---|---|
| * Yield of octene obtained per kg of nickel atoms (kg) | | | | |

### Reference Example 1

In a flask which had been completely dried with heating and nitrogen-substituted was put 0.366 g of anhydrous aluminum chloride, followed by heating to 100°C under reduced pressure (1 mmHg) to dry the aluminum chloride. Then, EADC (a 20 wt% solution in toluene, 20 ml, Nippon Aluminum Alkyls, Ltd.) was added thereto, followed by stirring at room temperature for 2 hours. After insoluble matters were sedimented, the supernatant was transferred into another completely heat-dried and nitrogen-substituted flask to obtain treated EADC.

In the same manner as above, different kinds of treated EADC were prepared by adding to EADC zirconium chloride, magnesium chloride, zinc chloride, sodium chloride, lanthanum chloride and calcium chloride, respectively, in place of aluminum chloride.

### Reference Example 2

A water separator equipped with a Dimroth condenser was attached to one of the necks of a two-neck flask and the other neck was stoppered with a rubber septum. Toluene (30 ml) and 0.732 g of aluminum chloride were put in the flask. The flask was heated in an oil bath to toluene reflux and the water separated by the water separator was discharged from the system to obtain a dispersed solution of dry aluminum chloride in toluene. The obtained dispersed solution was cooled to room temperature and EADC (a 50 wt% solution in toluene, 10 ml, Nippon Aluminum Alkyls, Ltd.) was added thereto through the neck stoppered with the rubber septum, followed by stirring at room temperature for 2 hours. After insoluble matters were sedimented, the supernatant was transferred into a completely heat-dried and nitrogen-substituted flask to obtain treated EADC.

In the same manner as above, different kinds of treated EADC were prepared by adding to EADC zirconium chloride, magnesium chloride, zinc chloride, sodium chloride, lanthanum chloride and calcium chloride, respectively, in place of aluminum chloride.

### Reference Example 3

The same procedure as in Reference Example 1 or 2 was repeated using lithium chloride to obtain treated EADC. As no insoluble matter was formed in this case, the treated EADC obtained was applied to dimerization reaction as such.

### Industrial Applicability

The present invention provides a process for dimerizing lower olefins with very high yield and selectivity and also with very high yield per unit weight of catalyst. Also provided is a catalyst to be used in said process.

## Claims

1. A process for dimerizing lower olefins which is characterized by the use of a catalyst comprising the following three components: (1) at least one salt or complex of a transition metal belonging to the platinum group of the periodic table, (2) at least one organoaluminum compound and (3) at least one inorganic metallic compound.

2. The process according to Claim 1, wherein said dimerization is carried out in a homogeneous reaction system by the use of the catalyst which is soluble in the lower olefins.

3. The process according to Claim 1, wherein the amount of said organoaluminum compound is in the range of 5 to 30 in the molar ratio based on that of said salt or complex of a transition metal belonging to the platinum group of the periodic table.

4. The process according to Claim 2, wherein the amount of said organoaluminum compound is in the range of 5 to 30 in the molar ratio based on that of said salt or complex of a transition metal belonging to the platinum group of the periodic table.

5. The process according to any of Claims 1-4, wherein said transition metal belonging to the platinum group of the periodic table is nickel.

6. The process according to Claim 5, wherein said salt or complex of the transition metal belonging to the platinum group of the periodic table is nickel 2-ethylhexanoate.

7. The process according to any of Claims 1-4, wherein said organoaluminum compound is selected from the group consisting of trialkylaluminum, dialkylaluminum halides and alkylaluminum dihalides.

8. The process according to Claim 7, wherein said organoaluminum compound is ethylaluminum dichloride.

9. The process according to any of Claims 1-4, wherein said inorganic metallic compound is selected from the group consisting of halides of aluminum, titanium, zirconium, magnesium, zinc, sodium, lithium, lanthanum and calcium.

10. The process according to Claim 9, wherein said inorganic metallic compound is selected from the group consisting of aluminum chloride, magnesium chloride, zinc chloride, lithium chloride and calcium chloride.

11. The process according to Claim 10, wherein said salt or complex of a transition metal belonging to the platinum group of the periodic table is nickel 2-ethylhexanoate, and said organoaluminum compound is ethylaluminum dichloride.

12. A catalyst which is obtainable by mixing (1) at least one salt or complex of a transition metal belonging to the platinum group of the periodic cable, (2) at least one organoaluminum compound and (3) at least one inorganic metallic compound.

13. The catalyst according to Claim 12, said catalyst being soluble in lower olefins.

14. The catalyst according to Claim 12, wherein the amount of said organoaluminum compound is in the range of 5 to 30 in the molar ratio based on that of said salt or complex of a transition metal belonging to the platinum group of the periodic table.

15. The catalyst according to Claim 13, wherein the amount of said organoaluminum compound is in the range of 5 to 30 in the molar ratio based on that of said salt or complex of a transition metal belonging to the platinum group of the periodic table.

16. The catalyst according to any of Claims 12-15, wherein said transition metal belonging to the platinum group of the periodic table is nickel.

17. The catalyst according to Claim 16, wherein said salt or complex of the transition metal belonging to the platinum group of the periodic table is nickel 2-ethylhexanoate.

18. The catalyst according to any of Claims 12-15, wherein said organoaluminum compound is selected from the group consisting of trialkylaluminum, dialkylaluminum halides and alkylaluminum dihalides.

19. The catalyst according to Claim 18, wherein said organoaluminum compound is ethylaluminum dichloride.

20. The catalyst according to any of Claims 12-15, wherein said inorganic metallic compound is selected from the group consisting of halides of aluminum, titanium, zirconium, magnesium, zinc, sodium, lithium, lanthanum and calcium.

21. The catalyst according to Claim 20, wherein said inorganic metallic compound is selected from the group consisting of aluminum chloride, magnesium chloride, zinc chloride, lithium chloride and calcium chloride.

22. The catalyst according to Claim 21, wherein said salt or complex of a transition metal belonging to the platinum group of the periodic table is nickel 2-ethylhexanoate, and said organoaluminum compound is ethylaluminum dichloride.
